# EUROPEAN PATENT APPLICATION

(11) **EP 2 923 713 A1**
(43) Date of publication of application: **30.09.2015**
(21) Application number: 15159393.6
(22) Date of filing: 17.03.2015
(51) Int. Cl.: A61M 1/00, A61M 1/08

(54) **Aspirator for aspirating venom or poison subsequent to a bite or sting**

(30) Priority: 24.03.2014 DK 201470142; 06.11.2014 DK 201470676
(71) Applicant: Brodsgaard Hessel, Sanne, 7700 Thisted (DK)
(72) Inventor: Brodsgaard Hessel, Sanne, 7700 Thisted (DK)
(74) Representative: Holme Patent A/S

(57) **Abstract**

An aspirator (1) comprising an elongated housing (2), a piston member (3) and a grip (15a,15b) protruding through at least one slot (9a,9b) of a circumferential wall of the housing (2) for moving the piston member (3) axially to adjust aspiration pressure, and an end closure (5) for confining the piston member (3) inside the housing (2). The aspirator has a suction end (7) partly closed by an annular end wall (11) inclining inside the housing (2) and encircling the suction opening (16) to form a contact surface against the skin that enables fast and effective sealing during retraction of the piston member to create a negative pressure.

## Description

The present invention relates to an aspirator comprising
- an elongated housing defined by a circumferential wall delimiting a suction chamber,
- the housing has a suction end with a suction opening and an opposite end,
- the circumferential wall has at least one slot extending axially from the opposite end a distance towards the suction end,
- a piston member comprising a piston head connected to a piston rod, which piston head and piston rod are movably between a fully depressed position and a fully retracted position inside the housing, and a grip protruding through the at least one slot of the housing for moving the piston member axially inside the housing to adjust aspiration pressure, and
- an end closure for closing the opposite end of the housing to confine the piston member inside the housing.

Stings and bites from insects, such as bees, wasps, hornets, ants and mosquitoes, or even bites from spiders, scorpions and snakes, transfer substances to the poor victim causing numerous inconveniences, including but not limited to irritation, redness, itching and/or swelling, and may even trigger a dangerous allergic reaction. An allergic reaction may cause an anaphylactic shock critical to the individual's health, and even result in death. Similarly can happen upon contact with poisonous plants and bushes, as well as with fish and marine animals, e.g. sea urchins, weevers and sea scorpions. This list is not exhaustive.

Various treatments of the above symptoms and the immunological reactions and conditions are available depending on the type of reactions. Redness and pain may be treated with local application of ice, pain-killers, and simply avoiding scratching. Antihistamine taken orally or applied locally also helps to suppress symptoms. Some lethal venoms and severe conditions may require immediate treatment with one or more of an epinephrine injection, tourniquet, and antihistamine. Medical treatment may also include drugs such as diphenhydramine and steroids.

Also available on the commercial market are mechanical syringe-like devices for aspirating substances placed under the skin by e.g. an insect due to a sting or bite. Use of such devices as fast as possible after the incident to extract the substance the insect transferred by biting or stinging may limit the individual's response and reactions to the substance considerably.

One example of such a known mechanical syringe-like invenomization device is known under the trademark ASPIVENIN® and described in US patent no. US 4,287,819A. This known device is a double acting twin-chamber mini-pump, which can create a vacuum directly over bites or stings to extract venom to relieve or avoid or reduce unhealthy responses and reactions to the venom. The device comes with a selection of various tip adaptors, albeit with same nozzle size. With the plunger in retracted position the selected tip adaptor is directed above the wound resulting from the bite or sting. Then the plunger is pushed fully inside the barrel chamber without lifting the nozzle from the skin, and this way a vacuum builds up that withdraw the venom. When the operator is satisfied that venom has been aspirated, the plunger is withdrawn to release pressure. Aspirate is confined inside the device. Operation of this device is however cumbersome and tricky. Withdrawal of the piston, and thus relief of pressure, must e.g. be performed using the thumb on the hand that holds the device, or using the other hand to pull the piston back and away from the site of use, thus a rather long distance. Operation may even require help from another person. A further disadvantage is the protruding pointed syringe tip, which only provide for a very limited engagement surface between skin and device, resulting in that the device often falls off prior to having ended the operation succesfully. The pointed tip is pressed into the wound under the influence of the created negative pressure, and this is both uncomfortable and painful to the individual.

An alternative device, which is simpler and easier to operate is the Poison Extractor® obtainable from PCG ApS, Thyrasvej 8, DK 2800 Rungsted Kyst. In this device vacuum is created upon withdrawal of a plunger confined in a housing. In contrast to the small nozzle opening of the ASPIVENIN® device, the Poison Extractor® has a mouth-piece with a large opening surrounded by a collar for surrounding the wound to better extract venom. The mouth-piece may be turned upside down so that the nozzle protrudes as for a conventional syringe. The plunger has two projecting grips substantially perpendicular to the longitudinal axis of the plunger and being secured to the plunger for withdrawal of same in order to create the required vacuum. One disadvantage of this known device is however that due to the large opening of the mouth-piece vacuum builds up too slow, and to an unsatisfactory extent, causing unreliable attachment to the skin and inefficient extraction of venom. After elapse of a selected extraction period the engagement between and device is relieved, e.g. by tilting the device in relation to the skin. The piston head of this known device has an O-ring of silicone to seal against the interior wall of the housing, but this O-ring dries out fast and after only a few piston strokes it displaces axially from it's seat on the piston head making the device inoperable.

None of the above prior art devices provides a satisfactory mechanical means for prompt and efficient aspiration and extraction of venom and other undesired substances from an individual's body, such as from under the skin or an infected local area, and there is a need and demand for alternative devices.

In a first aspect according to the present invention is provided an aspirator of the kind mentioned in the opening paragraph which is simple in structure, and efficient to operate for extraction of venom or poison transferred by an insect, animal or other object to an individual via a bite, sting or other interaction.

In a second aspect according to the present invention is provided an aspirator of the kind mentioned in the opening paragraph by means of which inconveniences, such as itching, pain, stress, fear, redness, and immunological reactions resulting from venom, poison or toxin transferred by an insect, animal or other object to an individual via a bite, sting or other interaction can be relieved, reduced or prevented.

In a third aspect according to the present invention is provided an aspirator of the kind mentioned in the opening paragraph which allows the operator to monitor aspiration of venom, poison or toxin transferred by an insect, animal or other object to an individual via a bite, sting or other interaction.

In a fourth aspect according to the present invention is provided an aspirator of the kind mentioned in the opening paragraph which is easy and fast to operate for aspiration of venom, poison or toxin transferred by an insect, animal or other object to an individual via a bite, sting or other interaction.

The novel and unique whereby these and other aspects are achieved according to the invention consists in that the suction end of the elongated housing is partly closed by an annular end wall inclining inside the housing and encircling the suction opening.

Within the context of the present invention the term "aspirator" means a device suited to create, increase, decrease, slow, stop or "pulse" aspiration force, in particular apply a negative pressure to a local site or spot. The aspirator thus produces suction to move or collect matter by suction and extraction.

By providing the annular end wall with an inclination the suction end of the aspirator fits better on top of a wound, in particular on top of a swollen wound. A sufficiently tight contact and closure between skin surface and suction end to create a negative pressure when the piston head is pulled away from the wound by pulling the grips axially thereby displacing the piston head is thus facilitated. The inclined annular end wall also serves to position the suction opening inside the housing. How far the suction opening is positioned inside the housing may vary and depends on the inclination angle.

The annular end wall preferably extends as a tapering funnel inside the housing. This design of the annular end wall is then a conical part having the smallest diameter, the suction opening, facing inside the housing to cup a wound when applied on top of said wound. The suction opening is in this manner retracted from the outmost end face of the suction end but is inverted compared to the conventional devices, which conventional devices have nozzles exterior to the housing. So the inclined annular end wall extends from the perimeter of the housing at the suction end.

Optionally the inclined annular end wall extends via a step that is substantially perpendicular to the circumferential wall or the step is less inclined than the inmost part of the annular end wall to have a more flat part for contacting the skin around the wound. Upon aspiration by withdrawal of the piston member the fluid substance is forced to flows from below the skin via the large inlet opening of the funnel further into the housing via the narrow diameter at the suction opening. The partial restriction of diameter causes a pressure difference that causes the aspirated fluid substance to accelerate towards the low narrow section on the other side of the suction opening inside the suction chamber of the housing, in which suction chamber the aspirated fluid substance may be captured.

The inclination of the annular end wall may differ to obtain various embodiments and designs. For example the angle between the annular end wall and the circumferential wall may be less than 90°, alternatively less than or equal to 80°, alternative less than or equal to 70°, alternative less than or equal to 60°, alternative less than or equal to 50°, alternative less than or equal to 40°. The annular end wall may include annular steps or annular sections of different angles.

In one embodiment the piston head has a cavity surrounding the annular end wall when the piston head is in the fully depressed position inside the housing. In this way is provided the longest possible piston stroke for application of negative pressure to the wound, as well as increasing accessible entire interior space of the housing, thus the volume of the suction chamber is maximum given the presence of the piston member and the closure end topping the housing to restrict reciprocation of the piston member. The cavity also serves to improve aspiration properties, such as acting as part of the suction chamber on the side of the suction opening opposite the annular end wall during retraction of the piston member. The cavity may also serve to collect initial aspirate.

The piston head may comprise at least one sealing ring that may surround the piston head and being dimensioned to engage the interior of the circumferential wall of the housing to prevent any leakage between circumferential wall and piston head that might comprise creation of negative pressure upon pulling of the piston head away from the suction end.

This sealing ring must have good sliding and sealing properties. It must also be durable and able to resist wear due axial displacement, and it must stay put on the piston head in response to reciprocating said piston head.

The at least one sealing ring can e.g. be made of a material selected from the group comprising thermoplastic elastomers or rubbers, or combinations thereof. A suitable greasing or lubricant may be added to prevent the at least one sealing ring from getting stuck to the interior circumferential wall when the piston is reciprocated, provided such means does not affect sealing properties. The thermoplastic elastomers or rubbers should be soft but not too soft, and of a kind that does not dry out easily. Examples are thermoplastic polyurethanes, polyacrylate, ethylene acrylate, butyl rubber, polychloroprene rubber, ethylene propylene rubber, flourosilicone, silicone rubber, acrylonitrile-butadiene rubber, hydrogenated nitrile rubber, fluorocarbon, tetrafluoroethylene-propylene, and flour elastomers.

The housing should be transparent to allow the operator to monitor the aspiration process. Known devices are not transparent, so the operator has no knowledge at all of whether aspiration is in progress, or if the device is used correctly and is working. Thus transparency is a means for verification and monitoring positive aspiration. In case the operator observes malfunction or lack of aspirate he/she can start over again without delay, thereby saving vital time.

The housing may preferably be made of a plastic material to which is added Armid®, commercially obtainable from AkzoNobel, or another chemical additive, agent or component having similar properties, added to reduce friction between interior wall and sealing ring. Optionally also the sealing ring can include such friction reducing material. Such other chemical additive cause the piston to reciprocate smoothly while still keeping leaktight contact with the interior wall of the housing upon reciprocating the piston member.

An aspirator having a housing with two opposite slots for receiving two corresponding axially movable protruding grips having free ends is very easy to operate. In use the opposite grips slide in corresponding lengthwise slots to serve as a dual grip of a handle to stabilize use and ease manipulation.

In one embodiment a grip can be curved so that a first free end of the grip is closer to the end closure, when viewed along the longitudinal axis of the housing, than an opposite second end attached to the piston rod, whereby one or more fingers easily can be folded beneath the curvature of the grips to grasp said grips. The curvature may be a section of a circle.

In one embodiment the annular end wall has a tubular end piece facing inside the housing. The tubular end piece may advantageously be dimensioned to pass into the cavity of the piston head.

The inclining annular end wall of the aspirator confers to the suction end an optimum contact surface against the skin that enables fast and effective sealing during retraction of the piston member during creating the negative pressure.

For some uses where the sting or bite is hard to access, such as in cavities or hairy locations, the aspirator may further comprise a suction tip having a coupling end opposite a suction tip end. The coupling end may advantageously have a socket fitting into the suction opening of the aspirator to allow passage into the elongated housing upon pulling the piston member backwards. So the socket mates fluid-tight into the suction opening to prevent false air entering, which would comprises suction efficiency. An annular wall delimiting a suction channel serves to put the suction opening in fluid communication with the suction channel, which suction channel has a suction inlet at the suction tip end and a suction outlet at the socket. The suction tip provides an elongation of the aspirator to facilitate its use in awkward positions and difficult locations, such being e.g. the hairy scalp or inside an ear.

The versatility of the aspirator is highly enhanced in that the suction tip is detachable from the elongated housing.

The annular wall of the suction tip may taper towards the suction tip end, optionally by the exterior face of the annular wall being incurved towards the suction channel. This design is convenient to e.g. spread apart hairs on the injury site so that optimum placing of the suction inlet above the relevant site, preferably surrounding the relevant poisoned lesion as much as possible for the best aspiration to be performed.

The flow of poison may benefit form the diameter of the suction channel at the suction inlet being smaller than at the suction outlet.

The aspirator can be used for aspiration of venom or poison subsequent to a bite or sting on both an animal or a human, adult or child, hairy skin or smooth skin.

The invention will now be described by way of an exemplary embodiment with references to the accompanying drawing in which,
Fig. 1 is a perspective view of the main components of an aspirator according to the present invention,
Fig. 2 shows the same in an assembled state where the piston member is fully depressed.
Fig. 3 is a perspective view of the housing seen from the bottom of the suction end,
Fig. 4 is a longitudinal sectional view taken along line IV-IV in fig. 3, but seen slightly from the opposite end of the housing,
Fig. 5 is an enlarged scale view of a fragment of the suction end of the housing,
Fig. 6 is a perspective view of the piston seen from the bottom of the piston head,
Fig. 7 is a longitudinal sectional fragmentary view taken along line VII-VII in fig. 6 of the piston head,
Fig. 8 is the aspirator in an intermediate aspiration step,
Fig. 9 is the aspirator at the end of an aspiration step and at the end of a backwards piston stroke,
Fig. 10 is an exploded view of the aspirator with a suction tip,
Fig. 11 is a sectional view along the longitudinal axis of the suction tip seen in fig. 11, and
Fig. 12 is a graph showing the suction pressure versus time of the aspirator during use.

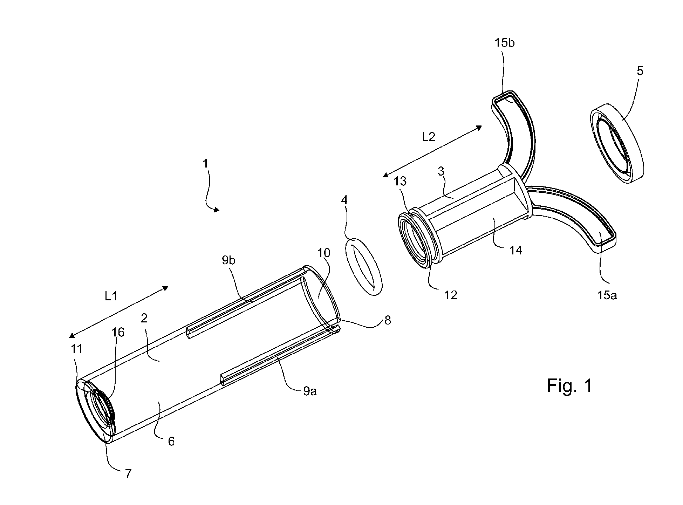

Fig. 1 is an exploded view of an embodiment of an aspirator 1. The aspirator 1 has a housing 2, a piston member 3, a sealing ring 4 and an end closure 5.

The housing 2 is in the present case cylindrical but other cross-sections than circular may be possible provided the piston member 3 is designed to reciprocate inside the housing 2 to create a vacuum upon retraction.

The housing 2 has a circumferential wall 6, a suction end 7 and an opposite end 8. The circumferential wall 6 has two opposites slots 9a,9b extending axially along the length of the housing from the opposite end 8 and a distance d towards the suction end 7. The piston member 3 is introduced inside the housing 2 via the opening 10 of the opposite end 8 of the housing 2. The design of an annular end wall 11 of the suction end 7 will be described in more detailed in the further figures.

The piston member 3 has a piston head 12, with an annular recess 13 for receiving the sealing ring 4. The piston head 12 extends in a piston rod 14, which piston rod 14 bifurcates into two opposite grips 15a,15b that curves outward and away from each other away from the piston head 12. In the present embodiment the curvature of the grips are substantially circular. The grips 15a,15b are flat to allow them to slide in the slots 9a,9b of the housing 2. In the present embodiment the grips 15a,15b are flat along the entire length but the part of the grips 15a,15b intended for being exterior to the housing 2, when the aspirator is assembled, may have a different shape. By the term flat is meant a shape and thickness not thicker than the gap of a slot 9a,9b. The axial length L2 of the piston rod 14 and the piston head 12 corresponds substantially to the interior axial lengths L1 of the housing from it's bottom at the suction end 7 to the end of the slots 9a,9b.

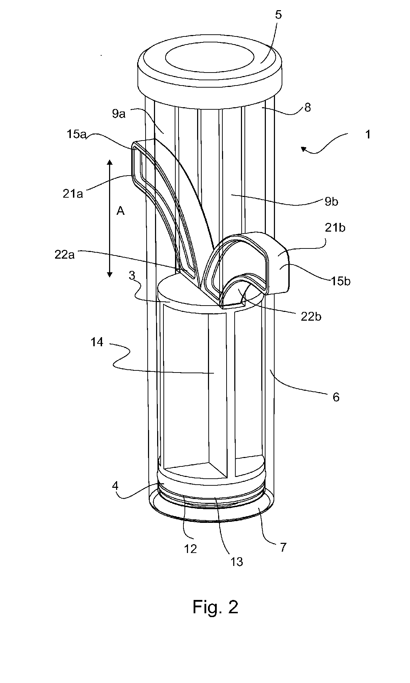

When the aspirator 1 is in assembled state seen in fig. 2, where the piston member 3 is inserted in the housing 2, and the end closure 5 is firmly secured on top of the opposite end 8 of the housing 2, to confine the piston member 3 inside said housing 2 and delimit the travel of a piston stroke as well as a suction chamber. The end closure 5 is the upper stop for the reciprocating of the piston member 3, as indicated by double arrow A. The suction opening 16 defines an orifice for creation of a negative pressure when the piston member 3 is pulled away from the suction end 7 in use of the aspirator 1, thus when the annular end wall 11 is placed on the intended surface wherefrom aspiration is to take place, such as a surface as the skin.

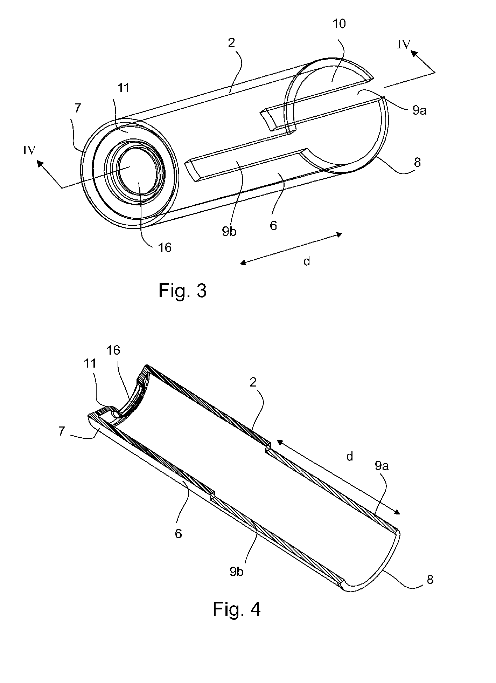

Fig. 3 shows the housing 3 in perspective, and in fig. 4 the housing is seen along a sectional view taken along line IV-IV in fig. 3, to illustrate the funnel-shape of the annular end wall 11 and the orifice of suction opening 16.

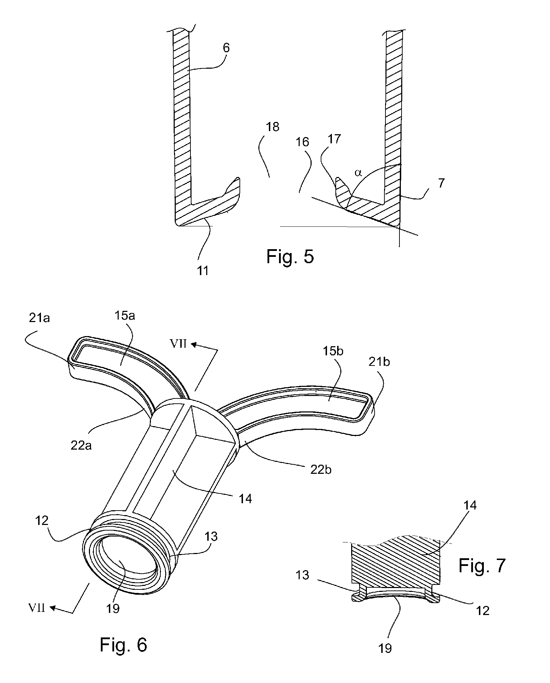

The inclination of the annular end wall is seen better in fig. 5, which is an enlarged scale, sectional view of the suction end 7. The annular end wall 11 extends from the free end of the cylindrical wall 6 of the suction end 7 via an angle α smaller than 90° and extends into a tubular piece 17 substantially concentric with the circumferential wall 6 to delimit the orifice 18 of the suction opening 16, and thus providing an aspiration nozzle.

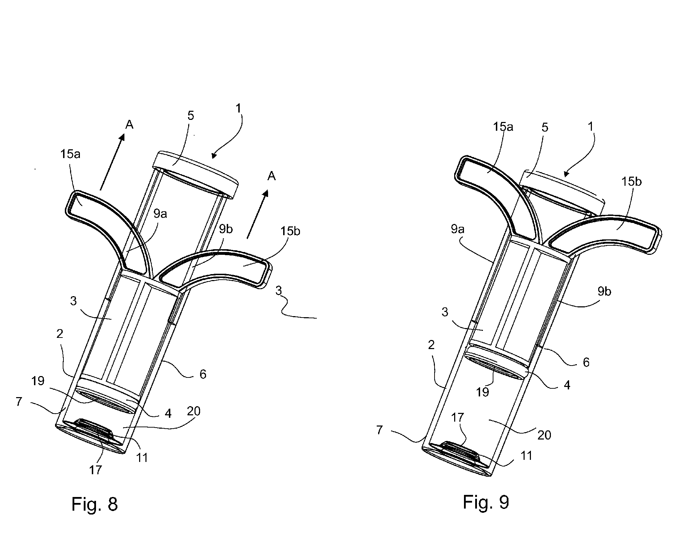

Fig. 6 is a perspective view of the piston member 3 seen from the bottom of the piston head 12 illustrating the hollow piston head 12, thus that the piston head 12 has a internal cavity 19 that in the depressed position of the piston head 12 seen in fig. 2, at least partly accommodates the annular end wall 11 of the suction end, such as accommodating the tubular piece 17 and in the retracted positions seen in figs. 8 and 9 add volume to the suction chamber 20 of the housing 3. The grips 15a,15b have first free end 21a,21b and extends via a bend curvature into second opposite ends 22a,22b firmly secured to the end of the piston rod opposite the cavity 19.

The internal cavity 19 is seen better in the fragmentary view of fig. 7, which is a longitudinal sectional view of the piston head 12 of fig 6. The internal cavity 19 extends axially and radially inside the piston head 12, and is surrounded by the proximal end of the piston head 12 having the recess 13 for the sealing ring 4, an O-ring 4, which O-ring 4 has a diameter corresponding to the height of the recess 13 or larger to be engaged properly inside the recess 13 to avoid displacement when the piston head 12 reciprocates.

In fig. 8 the aspirator 1 is in an intermediate aspiration step towards the end position seen in fig. 9, where the grips 15a,15b are stopped by the end closure 5 and prevented from further backwards movement. Thus the end closure 5 defines the outmost backward position of the piston head 12.

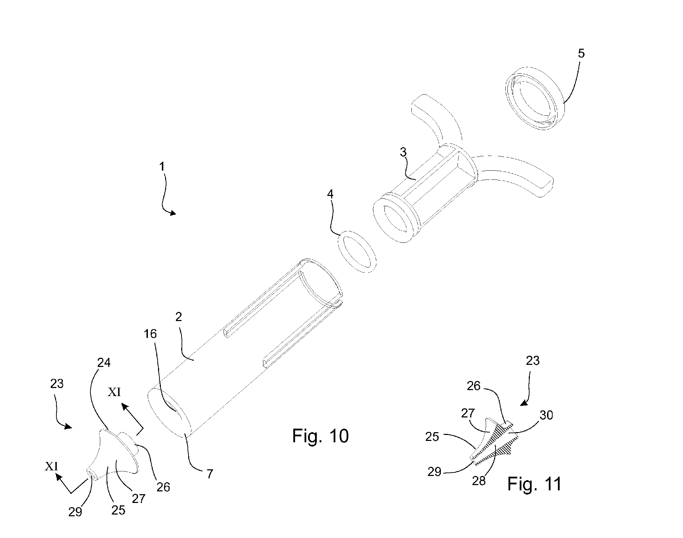

The suction tip 23 seen in the exploded view of fig. 10 has a coupling end 24 opposite a suction tip end 25. The coupling end 24 has a socket 26 fitting into the suction opening 16 of the aspirator 1. An annular wall 27 delimits a suction channel 28 in communication with the suction opening 16, which annular wall 27 is incurved against the suction channel 28. The socket 26 mates into suction opening 16 to put the suction tip 23 in fluid communication with the interior of the elongated housing 2 via the suction opening of the aspirator 1. The suction channel has 28 has a suction inlet 29 at the suction tip end 25 and a suction outlet 30 in the socket 26. The suction tip end 25 is the mouth placed at the site where aspiration must be made. To promote rapid aspiration the diameter of the suction channel 28 at the suction inlet 29 is smaller than at the suction outlet 30.

The incurved annular wall 27 and the suction channel 28 of the suction tip 23 is seen better in the sectional view of fig. 11. The aspirator 1 according to the present invention can be reused after cleaning or be disposed. The components for the aspirator 1 can all be made using conventional molding techniques, including injection molding.

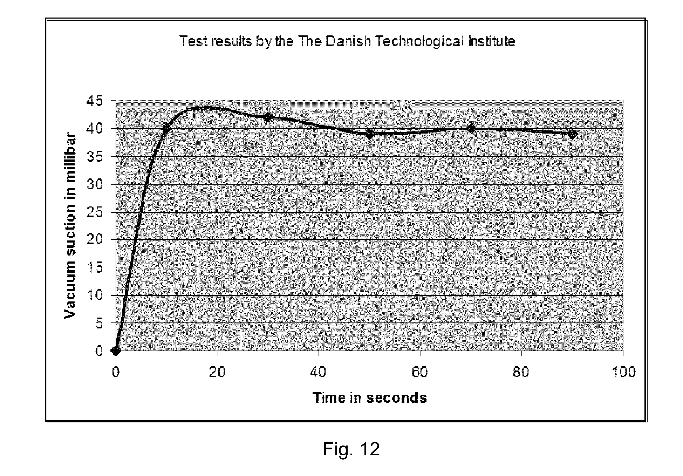

Fig. 12 is a graph showing that the aspirator according to the invention maintains a high level of suction, which is highly constant. The aspirator used for the test was may in accordance with the below otherwise non-limiting production example.

### Production example

End closure, piston member and suction tip was manufactured of Polypropylene type RF830 MO from Borealis, Vienna, Austria with an additive of 2% red color type PEZ143780X-Reed from Clariant, Odense C, Denmark. Color is not mandatory and other colors can be used instead of red.

The elongated housing was manufactured of Polypropylene type RF830 MO from Borealis, Vienna, Austria with an additive of 0,3% Armid type PUD18065-Natur.

The sealing ring was made of SILICONE RUBBER, SILICA model No. NE 140 from SGS, Shanghai China.

In the alternative the above polypropylene can be replaced with Polypropylen (Homopolymer) type Purell HP371P.

The aspirator 1 according to the present invention permits local emergency extraction of the venom or poison from a bite or sting inoculated by a venomous animal. The aspirator 1 advantageously allows a user to suck strongly and easily by manual operation not only venom or poisons from under the skin due to an insect bite, but also pus due to infection of the skin or the like, and any other substance from a surface using a single hand in a rapid and simple operation. Several pistons strokes are possible but rarely needed due to the effective contact between surface, e.g. the skin surrounding the wound from the sting or bite, permitting a negative pressure to build up instantaneously to aspirate the undesired substance.

## Claims

1. An aspirator (1) comprising
- an elongated housing (2) defined by a circumferential wall (6) delimiting a suction chamber (20),
- the housing (2) has a suction end (7) with a suction opening (16) and an opposite end (8),
- the circumferential wall (6) has at least one slot (9a,9b) extending axially from the opposite end (8) a distance (d) towards the suction end (7),
- a piston member (3) comprising a piston head (12) connected to a piston rod (14), which piston head (12) and piston rod (14) are movably between a fully depressed position and a fully retracted position inside the housing (2), and a grip (15a,15b) protruding through the at least one slot (9a,9b) of the housing (2) for moving the piston member (3) axially inside the housing (2) to adjust aspiration pressure, and
- an end closure (5) for closing the opposite end (8) of the housing (2) to confine the piston member (3) inside the housing (2),
**characterised in that**
- the suction end (7) of the elongated housing (2) is partly closed by an annular end wall (11) inclining inside the housing (2) and encircling the suction opening (16).

2. An aspirator (1) according to claim 1, **characterised in that** the annular end wall (11) extends as a tapering funnel inside the housing (2).

3. An aspirator according to any of claims 1 or 2, **characterised in that** the inclining of the annular end wall (11) is at an angle (α) between the annular end wall and the circumferential wall of the housing (2), which angle (α) is less than 90°, alternatively less than or equal to 80°, alternative less than or equal to 70°, alternative less than or equal to 60°, alternative less than or equal to 50°, alternative less than or equal to 40°.

4. An aspirator (1) according to any of the preceding claims 1, 2 or 3, **characterised in that** the piston head (12) has a cavity (19) surrounding the annular end wall (11) when the piston head (12) is in the fully depressed position inside the housing (2).

5. An aspirator (1) according to any of the preceding claim 1 - 4, **characterised in that** the piston head (12) comprises at least one sealing ring (4) surrounding the piston head (12) and dimensioned to engage the interior surface of the circumferential wall (6) of the housing (2).

6. An aspirator (1) according to claim 5, **characterised in that** the at least one sealing ring (4) is made of a material selected from the group comprising thermoplastic elastomers or rubbers, or combinations thereof.

7. An aspirator (1) according to any of the preceding claims 1 - 6, **characterised in that** the housing (2) is transparent.

8. An aspirator (1) according to any of the preceding claims 1 - 7, **characterised in that** the housing (2) has two opposite slots (9a,9b) for receiving two corresponding axially movable protruding grips (15a,15b) having free ends.

9. An aspirator (1) according to any of the preceding claims 1 - 8, **characterised in that** the grip (15a,15b) is curved so that a first free end of the grip (15a,15b) is closer to the end closure (5) than an opposite second end attached to the piston rod (14).

10. An aspirator (1) according to any of the preceding claims 1 - 9, **characterised in that** the annular end wall (11) has a tubular end piece (17) facing inside the housing (2).

11. An aspirator (1) according to any of the preceding claims 1 - 10, **characterised in that** the aspirator further comprises a suction tip (23) having
- a coupling end (24) opposite a suction tip end (25),
- the coupling end (24) has a socket (26) fitting into the suction opening (16) of the aspirator (1), and
- an annular wall (27) delimiting a suction channel (28) in communication with the suction opening (16), said suction channel (28) having a suction inlet (29) at the suction tip end (25) and a suction outlet (30) at the socket (26).

12. An aspirator (1) according to claim 11 **characterised in that** the suction tip (23) is detachable.

13. An aspirator (1) according to any of the preceding claims 1 - 12, **characterised in that** the annular wall (27) of the suction tip (23) tapers towards the suction tip end (25), optionally the exterior face of the annular wall (27) is incurved towards the suction channel (28).

14. An aspirator (1) according to any of the preceding claims 1 - 13, **characterised in that** the diameter of the suction channel (28) at the suction inlet (29) is smaller than at the suction outlet 30.

15. Use of the aspirator (1) according to any of the preceding claims 1 - 14 for aspiration of venom or poison subsequent to a bite or sting on an animal or a human.
